# EUROPEAN PATENT SPECIFICATION

(11) **EP 0 929 695 B1**
(45) Date of publication and mention of the grant of the patent: **09.10.2002**
(21) Application number: 97932935.6
(22) Date of filing: 24.07.1997
(51) Int. Cl.: C12Q 1/68, C07K 14/32

(54) **DETECTION OF PSYCHROTROPHIC BACILLUS**
BESTIMMUNG VON PSYCHOTROPISCHEM BACILLUS
DETECTION DU BACILLE PSYCHROTROPHE

(30) Priority: 24.07.1996 GB 9615516
(43) Date of publication of application: 21.07.1999
(73) Proprietor: Biotec Laboratories Limited, Ipswich, Suffolk IP5 7RG (GB)
(72) Inventor: STEWART, Gordon Sydney Anderson Birnie, Loughborough, Leicestershire LE11 0QL (GB); FRANCIS, Kevin Patrick, Lincoln, Lincolnshire LN6 9RR (GB); SCHERER, Siegfried, D-85354 Freising (DE); LECHNER, Sabine, D-97234 Reichenberg (DE); MAYR, Ralf, D-85356 Freising (DE)
(74) Representative: Wilkinson, Stephen John
(86) International application number: GB9701987
(87) International publication number: WO98003678

(56) References cited:
- EP-A- 0 395 292
- WO-A-96/15264
- MAYR ET AL.: "Identification and purification of a family of dimeric major cold shock protein homologs from the psychotropic bacillus cereus WSBC 10201" JOURNAL OF BACTERIOLOGY, vol. 178, no. 10, May 1996, pages 2916-2925, XP002047488
- SCHRÖDER ET AL.: "Mapping of the Bacillus subtilis cspB gene and cloning of its homologs in thermophilic, mesophilic and psychotropic bacilli" GENE, vol. 136, no. 1-2, 1993, pages 277-280, XP002047489

## Description

This invention concerns differentiating between strains of *Bacillus* (e.g. *B. cereus*), in particular between psychrotrophic and mesophilic strains.

*Bacillus cereus* and other *Bacillus* species such as *Bacillus mycoides* (previously known as *Bacillus cereus* var. *mycoides)* are bacteria associated with the spoilage of food products stored at refrigeration temperature. Spoilage of milk and other dairy products by this organism is a particular problem. The presence of *Bacillus cereus* in food products can be tested for by a well known biochemical test (API test) and affected food batches destroyed. However, this can be wasteful since certain strains of *Bacillus*, described as mesophilic, do not grow at refrigeration temperatures and therefore are not generally involved in spoilage of food stored at such temperatures. It is the psychrotrophic *Bacillus* strains, which do grow and proliferate at low temperatures such as the standard refrigeration temperature of 4 - 7°C, that will cause food to spoil. According to standard terminology, bacteria are psychrotrophic if they will grow and proliferate when left for ten days at 7°C or below.

Psychrotrophic bacteria have been recognised as a recurring problem in the refrigerated storage and distribution of fluid milk and other perishable dairy products. Although much emphasis has been focused on post-pasteurisation contaminants such as *Pseudomonas,* improved processing conditions for milk and other dairy products has meant less interest in these non-heat-resistant contaminants and more attention being directed at psychrotrophic sporeformers such as *Bacillus.* It has been estimated that 25% of all shelf-life problems associated with conventionally pasteurised milk and cream products in the USA may be linked to thermoduric psychrotrophs, with the majority of these contaminants being psychrotrophic *Bacillus* (e.g. *B. cereus*). Moreover, the incidence of psychrotrophic *Bacillus* species that are associated with the spoilage of dairy products is even greater in a number of European countries where the average storage temperature of many of these milk products is several degrees higher.

Therefore, it would be a significant advantage to have a test capable of distinguishing between psychrotrophic *Bacillus* and mesophilic *Bacillus.* The development of an assay capable of rapidly detecting and quantifying psychrotrophic *Bacillus* in milk and other dairy products would prove invaluable to the dairy industry globally. The value to the milk industry alone, worth £3.5 billion in the UK, would be very significant indeed. The present invention addresses these needs.

It has now been discovered that the DNA encoding the major cold shock protein homologue known as cspA in *Bacillus* shows conserved nucleotide differences, at particular positions in the sequence, between psychrotrophic and mesophilic strains.

The term "conserved nucleotide base difference" as used herein means a nucleotide base difference that occurs sufficiently consistently between psychrotrophic and mesophilic strains to provide the basis for a useful test for psychrotrophic *Bacillus.* A test for psychrotrophic *Bacillus* need not necessarily be 100% accurate to be useful, although clearly 100% accuracy would be preferable. For example, a base difference which occurs 90% or 95% of the time between psychotrophs and mesophils may provide the basis for a practical test. Preferably, any inconsistencies which occur are such that false positives rather than false negatives result, so that the presence of any psychotrophs in the sample being tested does not go undetected.

The invention therefore provides in one aspect a method of testing for a psychrotrophic *Bacillus* bacterium in a sample, which method comprises determining whether the sample contains a nucleic acid which codes for a *Bacillus* major cold shock protein or major cold shock protein homologue and which has at least one conserved nucleotide base which is different in psychrotrophic and mesophilic strains of *Bacillus.*

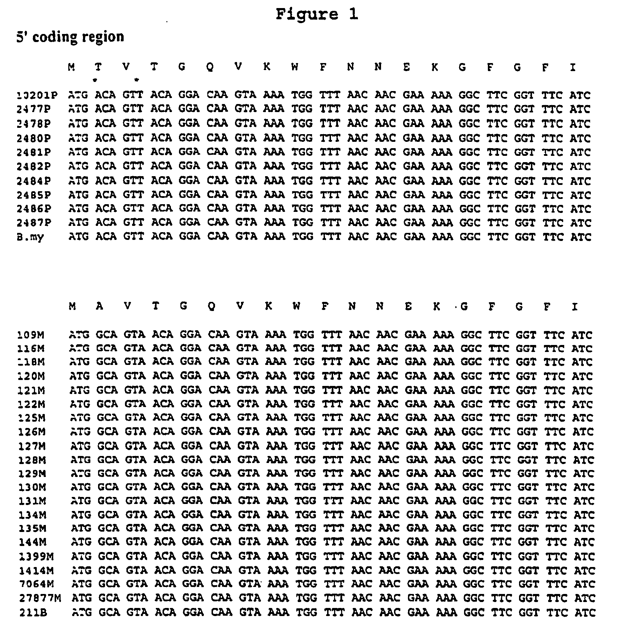

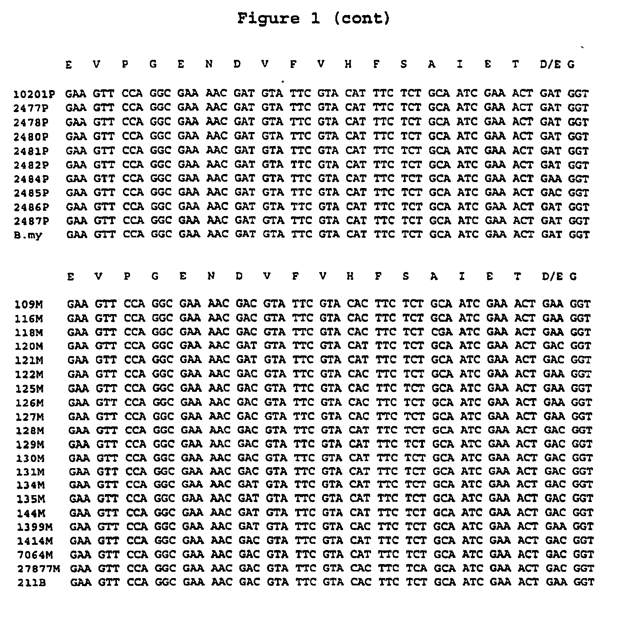

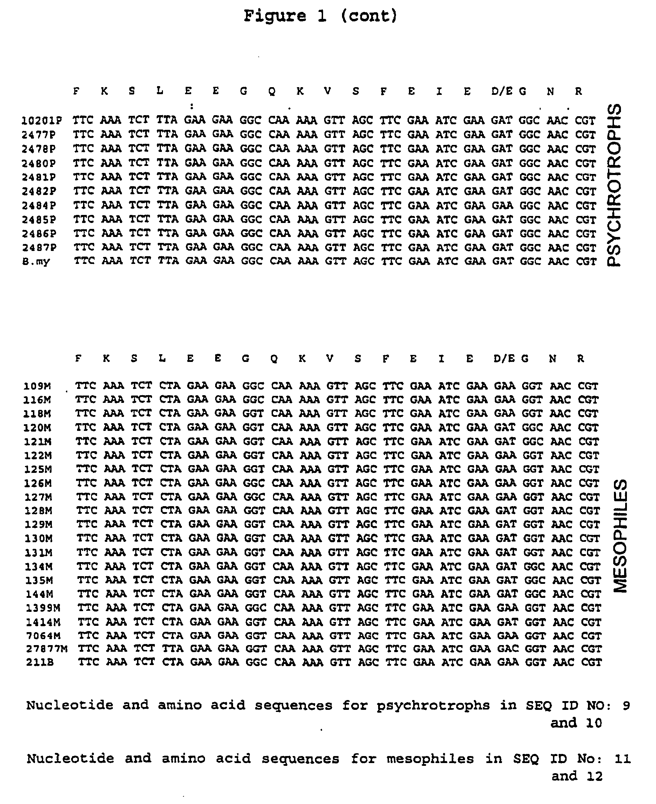

In a particular embodiment of the method according to the invention, the conserved nucleotide base is adenine (A) at position 4 or thymine (T) at position 9 in the nucleic acid sequence shown in Figure 1, which encodes the *Bacillus cereus* major cold shock protein homologue cspA, or both A at position 4 and T at position 9.

In another embodiment of the method according to the invention, the conserved nucleotide base is T at position 124 in the nucleic acid sequence shown in Figure 1, which encodes the *Bacillus cereus* major cold shock protein homologue cspA. It will be evident from Figure 1 that one of the sequenced mesophilic strains (designated 27877M) contains T at position 124 of the nucleotide sequence and would thus show up as a false positive in a test for psychrotrophic strains based on position 124. However, since strain 27877M represents only 1 out of the 21 mesophilic strains sequenced, position 124 is expected to provide a suitable basis for a test for psychrotrophic *Bacillus*.

In another aspect, the invention provides an oligonucleotide primer or probe, comprising at least 15 nucleotides, which is complementary to a region of the major cold shock protein DNA sequence for psychrotrophic *Bacillus cereus* shown in Figure 1, or to a region of its reverse complement, which region includes position 124 or position 4 or position 9, or both position 4 and position 9, in Figure 1.

The invention is thus concerned with methods for specifically detecting psychrotrophic *Bacillus,* and with oligonucleotides useful in such methods.

The invention is concerned primarily with detection of psychrotrophic organisms which are involved in food spoilage. The most commercially important of these is *Bacillus cereus,* but other *Bacillus* species such as *B. mycoides, B. circulans, B. coagulans, B. brevis and* *B. licheniformis* are also capable of proliferating at low temperatures and are relevant to some degree to food spoilage. Major cold shock protein homologues have been found to be highly conserved throughout a range of different bacteria in which they have been sought.

In the attached drawings;
Figure 1 shows the coding region of the major cold stock protein homologue cspA gene as sequenced from various strains of psychrotrophic and mesophilic *Bacillus cereus* and from a strain of psychrotrophic *Bacillus mycoides.* Part of the 3' end of the coding region, representing about 8 amino acids, is missing.
Figure 2 shows part of the non-coding region of the cspA gene upstream of the ATG at the 5' end of the Figure 1 sequence, for the same *Bacillus cereus* strains as Figure 1.
Figure 3 shows an ethidium bromide stained agarose gel in which PCR products from a screen for psychrotrophic *Bacillus cereus* have been run (see Example 3).

The variety of different techniques which may be applied in the method according to the invention will be known to those skilled in the art. Preferably, the method according to the invention uses analysis of nucleic acid sequences. However, analysis of protein encoded by the specific nucleic acid sequences is not excluded. The substitution of A in psychrotrophic *Bacillus* cereus strains by guanine (G) in mesophilic strains at position 4 in Figure 1 is a base change which results in a different amino acid being encoded by the codon which includes the nucleotide at position 4. Mesophiles have the second codon GCA which codes for alanine, whereas psychotrophs have the second codon ACA which codes for threonine. The A-G change is therefore a significant difference, particularly since the other base differences between psychrotrophic and mesophilic strains shown in Figure 1 do not represent amino acid alterations in the protein. Analysis of the protein sequence could be carried out by lysing the bacteria, isolating the cspA protein e.g. by means of a specific antibody directed against it, and N-terminal sequencing the protein to detect the amino acid sequence difference. Thus, differentiation between psychrotrophic and mesophilic *Bacillus* strains will also be possible at the amino acid level.

Generally, the method according to the invention will involve a nucleic acid amplification reaction. This may be performed on DNA or mRNA, or on cDNA produced from it. Amplification may be performed for example by the polymerase chain reaction (PCR). Determining whether a particular nucleic acid sequence is present may then be carried out by analysis of the amplification products, e.g. by sequencing, by the use of single-strand conformation polymorphism (SSCP) analysis, or by the use of a labelled probe which binds to the amplification reaction products. Alternatively, determining whether the specific sequence is present may be carried out as an integral part of the amplification reaction, e.g. using a probe cleavage amplification technique such as the system marketed by Perkin Elmer under the trade mark TaqMan. This is a particularly preferred technique for use in the methods according to the invention and is described in more detail below.

A preferred amplification technique for use in the method according to the invention is reverse transcriptase polymerase chain reaction (RT-PCR). RT-PCR uses the enzyme reverse transcriptase to convert mRNA to cDNA which then acts as a template for PCR. RT-PCR is advantageous over conventional PCR performed on sample DNA for two reasons. Firstly, false positives occurring due to the presence of non-viable bacteria are reduced. This is because mRNA should only be present in viable bacteria, whereas DNA is stably present in both dead and live bacteria. Secondly, major cold shock protein mRNA is highly induced at refrigeration temperatures, but inherently unstable at 37°C. This means that a sample can initially be incubated at 37°C to destroy residual template mRNA, followed by a short incubation at 15°C or below to allow transcription of the template mRNA from viable bacteria.

The system marketed by Perkin Elmer under the trade mark TaqMan is a PCR assay system and is preferably used in the method according to the invention in combination with RT-PCR. TaqMan™ uses the hydrolysis of a fluorogenic probe to monitor the extent of amplification. The probe consists of an oligonucleotide labelled with both a fluorescent reporter and a quencher dye. During the PCR process, this probe is cleaved by the 5' nuclease activity of taq DNA polymerase if and only if it hybridises to the segment being amplified. Cleavage of the probe generates an increase in the fluorescence intensity of the reporter dye. Thus, amplification of a specific product can be detected by simply measuring fluorescence after or during PCR.

An example of a suitable probe for specifically detecting psychrotrophic *Bacillus cereus* by a conserved nucleotide base difference at position 124 in Figure 1 is CAAATCTTTAGAAGAAGGCCAAAA [SEQ ID NO: 1]. This probe will hybridise to the reverse complement of the nucleotide sequence in Figure 1, from position 117 to 141. Under suitable conditions, this or a similar probe incorporating a conserved nucleotide base difference between psychrotrophic and mesophilic *Bacillus cereus* can be used to specifically detect psychrotrophic strains.

A particularly preferred probe for detecting psychrotrophic *Bacillus cereus* is one which hybridises in a TaqMan™ assay to a region of the nucleic acid in Figure 1, or a region of its reverse complement, which region includes positions 4 and 9. An example of such a probe is Psy 1

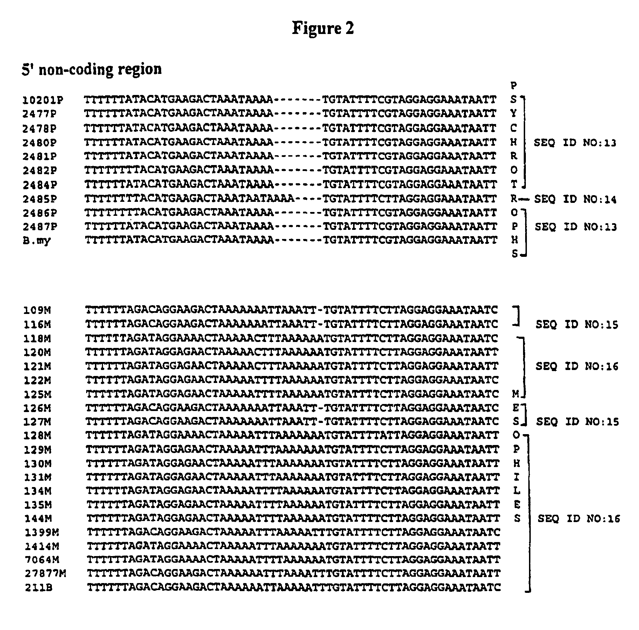

A suitable pair of primers for use in a TaqMan™ assay with the above probe is BcF2 and Bc57R or BcF2 and Bc56R with the following sequences: BcF2 is a forward primer which hybridises to a region upstream of the probe, to part of the non-coding region of the cspA gene upstream of the nucleotide sequence in Figure 2. Bc57R and Bc56R are reverse primers which hybridise downstream of the probe.

In addition to forward and reverse primers and a probe specific for psychrotrophic *Bacillus cereus,* a mesophile-specific probe may also be used. A suitable mesophile-specific probe for use in a TaqMan™ assay with the above probe and primers is Meso 1 which has the nucleotide sequence:

Psychrotroph- and mesophile-specific probes may be differently labelled and used together in a TaqMan™ assay. Each will have a different fluorescent reporter which can be separately detected in order to determine whether either or both types of *Bacillus* are present in a sample.

An alternative technique for direct screening for psychrotrophic *Bacillus* uses oligonucleotide primers incorporating the conserved nucleotide base differences found between psychrotrophic and mesophilic strains, in a conventional PCR reaction. The oligonucleotide primers are designed such that a conserved nucleotide difference is incorporated at the 3' end. At least one such primer, specific for psychrotrophic *Bacillus* sequences, can be used as one of a pair of primers in a conventional PCR reaction to detect psychrotrophs. Amplified products will only result in the presence of the target sequence. Preferably, both primers in the pair are psychrotroph-specific.

Suitable specific oligonucleotide primers incorporate the conserved nucleotide base difference at position 124, or 4, or 9 of the coding region of the cspA gene (see Figure 1) at the 3' end. For example, BcPF - 5' GGAAATAATTATGACAGTT 3' [SEQ ID NO:7] is specific for psychrotrophs and incorporates the conserved nucleotide base differences at both positions 4 and 9, position 9 being at the 3' end of the primer. BcPR - 5' CTTTTTGGCCTTCTTCTAA 3' [SEQ ID NO:8] incorporates position 124 of the sequence shown in Figure 1. The specific primers will be of a suitable length for the purpose of target specific amplification, for example between 10 and 30 nucleotides or between 15 and 25 nucleotides in length.

### EXAMPLES

### Example 1

### TaqMan™ screening of psychrotrophic and mesophilic Bacillus cereus

### PCR conditions

TaqMan™ PCR was performed in triplicate on 9 strains of psychrotrophic *B. cereus* (2478P, 2480P, 2482P, 2484P, 2485P, 2486P, 2487P, and 10201P) and 9 strains of mesophilic *B. cereus* (109M, 116M, 118M. 120M, 121M, 122M, 125M, 127M, and 128M), using the major cold-shock protein gene sequence *cspA* as a target. PCR was performed with a Perkin Elmer ABI 2400 automated thermocycler with 0.2 ml thin walled PCR tubes (Perkin Elmer). Reactions were carried out in 50 µl volumes containing 5µl of 10X PCR buffer (supplied with *Taq* DNA polymerase; Perkin Elmer), 4mM MgCl₂ (Perkin Elmer), 200 pmol of the oligonucleotide primers BcF2 (CGA ATT TGA TAA TGT GTG GAT TC [SEQ ID NO: 3]) and Bc57R (TCG CCT GGA ACT TCG ATG [SEQ ID NO: 4]), 100 pmol of the fluorogenic probes Meso 1 (TET- ATG GCA GTA ACA GGA CAA GTA AAA TGG T - TAMRA [SEQ ID NO: 6]) and Psy 1 (FAM - ATG ACA GTT ACA GGA CAA GTA AAA TGG TTT AAC - TAMRA [SEQ ID NO: 2]), 0.2 mM of each deoxynucleotide triphosphate (dATP, dCTP, dGTP, dTTP; Pharmacia), 1.25 U of *Taq* DNA polymerase (Perkin Elmer), and a pin head sized aliquot of bacteria picked from an agar plate of selective media. Bacterial cells were lysed by heating the mixture for 10 minutes at 95°C. Amplification of cspA DNA and probe cleavage was then attempted with 40 cycles at 95°C for 15 s and 62°C for 1 minute. Fluorescence was recorded from each PCR sample using a Perkin Elmer LS-50B (allelic discrimination mode).

### Results

Once the LS-50B had been calibrated using a no template control, samples were read as either Homo1 (psychrotrophic strain) or Homo2 (mesophilic strain). All 18 strains of *B. cereus* were called correctly.

### Example 2

### Isolation of Bacillus from milk

It has been demonstrated that subjecting milk to a "heat - shock" of 75°C for 20 min causes the vast majority of contaminating *Bacillus* spores to germinate. Hence the following procedure can be adopted to allow the isolation of *Bacillus* from milk:
1) Collect a sample of 100-200 ml of raw milk from the milk tanker, individual farm bulk tank, dairy holding tank etc. prior to pasteurisation.
2) Heat-shock the above sample(s) at 75°C for 20 min and then rapidly cool to 30°C - 35°C.
3) Incubate milk sample(s) at 30°C - 35°C for a period of 16 - 24 hours (time period and temperature should reflect conditions necessary to give between 10⁶ and 10⁸ bacteria per ml of milk).
4) Place 100 µl sample(s) of the above into a 1.5 ml microcentrifuge tube containing 900 µl of warm/hot (50°C - 60°C) washing diluent (e.g. sterile water containing a mild detergent such as Triton X-100).
5) Briefly vortex the solution and pellet bacterial cells at 13,000g for 10 min.
6) Repeat washing of bacterial cells with 1 ml of warm/hot washing diluent and re-pellet bacteria as in step 5.
7) Re-suspend bacterial pellet in 100 µl of sterile water and boil for 10 min to lyse bacteria.

### Quantification of Psychrotrophic Bacillus (B. cereus/mycoides)

1) Pipette 10 µl of cell lysate from above (step 7) into a thin walled PCR tube containing 90 µl of *B. cereus* master mix formulated as follows: 10 µl of 10X PCR buffer (supplied with *Taq* DNA polymerase), 4 mM MgCl₂, 200 pmol of the oligonucleotide primers BcF2 (CGA ATT TGA TAA TGT GTG GAT TC [SEQ ID NO:3]) and Bc57R (TCG CCT GGA ACT TCG ATG [SEQ ID NO: 4]), 100 pmol of the fluorogenic probe Psy 1 (FAM - ATG ACA GTT ACA GGA CAA GTA AAA TGG TTT AAC [SEQ ID NO:2]), 0.2 mM of each deoxynucleotide triphosphate (dATP, dCTP, dGTP, dTTP), and 1 U of *Taq* DNA polymerase.
2) Place PCR tube(s) in a thermal cycler and perform 40 cycles of 95°C for 15 s and 62°C for 1 min.
3) Use a real-time detector (e.g. ABI 7700) to monitor fluorescence and quantify starting template DNA.
4) Use above data to estimate the number of bacteria in the original sample and to estimate the quality/shelf life of the milk.

### Example 3

### Direct screening for psychrotrophic B.cereus using conventional PCR

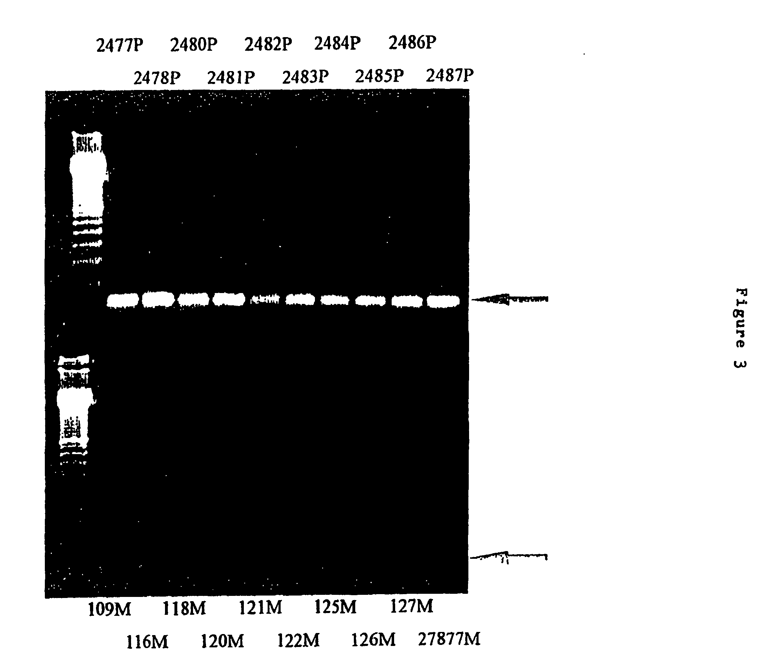

Oligonucleotide primers with psychrotrophic *B. cereus cspA* specific sequences (BcPF - 5' GGAAATAATTATGACAGTT 3' [SEQ ID NO: 7] and BcPR - 5' CTTTTTGGCCTTCTTCTAA 3' [SEQ ID NO: 8] were designed by incorporating the conserved nucleotide differences found between mesophilic and psychrotrophic strains of these bacteria (Figure 1). The latter primers were then used to perform PCR on genomic DNA from 10 mesophilic (109M, 116M, 118M, 120M, 121M, 122M, 125M, 127M, and 128M) and 10 psychrotrophic strains of *B. cereus* (2478P, 2480P, 2481P, 2482P, 2484P, 2485P, 2486P, 2487P, and 10201P). PCR was performed using 35 cycles of 95°C for 15 sec, 55°C for 30 sec, 72°C for 30 sec. The resulting PCR products were run on an ethidium bromide stained agarose gel (Figure 3). Only the group of 10 psychrotrophic strains gave amplified products, demonstrating the specificity of this assay for discriminating between mesophilic and psychrotrophic strains of *B. cereus.*

### SEQUENCE LISTING

(1) GENERAL INFORMATION:
   (i) APPLICANT:
      (A) NAME: Biotec Laboratories Limited
      (B) STREET: 32 Anson Road, Martlesham Heath
      (C) CITY: Ipswich
      (D) STATE: Suffolk
      (E) COUNTRY: United Kingdom
      (F) POSTAL CODE (ZIP): IP5 7RG
   (ii) TITLE OF INVENTION: Detection of Psychrotrophic Bacillus
   (iii) NUMBER OF SEQUENCES: 16
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (vi) PRIOR APPLICATION DATA:
      (A) APPLICATION NUMBER: GB 9615516.3
      (B) FILING DATE: 24-JUL-1996
(2) INFORMATION FOR SEQ ID NO: 1:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA probe"
      (xi) SEQUENCE DESCRIPTION: SEQ ID NO: 1:
   CAAATCTTTA GAAGAAGGCC AAAA 24
(2) INFORMATION FOR SEQ ID NO: 2:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA probe"
   (xi) SEQUENCE DESCRIPTION: SEQ ID NO: 2:
   ATGACAGTTA CAGGACAAGT AAAATGGTTT AAC 33
(2) INFORMATION FOR SEQ ID NO: 3:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA primer"
   (xi) SEQUENCE DESCRIPTION: SEQ ID NO: 3:
   CGAATTTGAT AATGTGTGGA TTC 23
(2) INFORMATION FOR SEQ ID NO: 4:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA primer"
   (xi) SEQUENCE DESCRIPTION: SEQ ID NO: 4:
   TCGCCTGGAA CTTCGATG 18
(2) INFORMATION FOR SEQ ID NO: 5:
   (i) SEQUENCE CHARACTERISTICS:
      (A) LENGTH: 18 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA primer"
   (xi) SEQUENCE DESCRIPTION: SEQ ID NO: 5:
   GCTTGAGGTC CACGGTTG 18
(2) INFORMATION FOR SEQ ID NO: 6:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA probe"
   (xi) SEQUENCE DESCRIPTION: SEQ ID NO: 6:
   ATGGCAGTAA CAGGACAAGT AAAATGGT 28
(2) INFORMATION FOR SEQ ID NO: 7:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA primer"
   (xi) SEQUENCE DESCRIPTION: SEQ ID NO: 7:
   GGAAATAATT ATGACAGTT 19
(2) INFORMATION FOR SEQ ID NO: 8:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: other nucleic acid
      (A) DESCRIPTION: /desc = "synthetic DNA primer"
   (xi) SEQUENCE DESCRIPTION: SEQ ID NO: 8:
   CTTTTTGGCC TTCTTCTAA                                                   19
(2) INFORMATION FOR SEQ ID NO: 9:
   (i) SEQUENCE CHARACTERISTICS:
      (A) LENGTH: 171 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:1..171
   (xi) SEQUENCE DESCRIPTION: SEQ ID NO: 9:
   ATG ACA GTT ACA GGA CAA GTA AAA TGG TTT AAC AAC GAA AAA GGC TTC      48
   Met Thr Val Thr Gly Gln Val Lys Trp Phe Asn Asn Glu Lys Gly Phe
     1               5                  10                  15
   GGT TTC ATC GAA GTT CCA GGC GAA AAC GAT GTA TTC GTA CAT TTC TCT      96
   Gly Phe Ile Glu Val Pro Gly Glu Asn Asp Val Phe Val His Phe Ser
                20                  25                  30
   GCA ATC GAA ACT GAH GGT TTC AAA TCT TTA GAA GAA GGC CAA AAA GTT     144
   Ala Ile Glu Thr Xaa Gly Phe Lys Ser Leu Glu Glu Gly Gln Lys Val
            35                  40                  45
   AGC TTC GAA ATC GAA GAT GGC AAC CGT                                 171
   Ser Phe Glu Ile Glu Asp Gly Asn Arg
        50                  55
(2) INFORMATION FOR SEQ ID NO: 10:
   (i) SEQUENCE CHARACTERISTICS:
      (A) LENGTH: 57 amino acids
      (B) TYPE: amino acid
      (D) TOPOLOGY: linear
   (ii) MOLECULE TYPE: protein
   (xi) SEQUENCE DESCRIPTION: SEQ ID NO: 10:
   Met Thr Val Thr Gly Gln Val Lys Trp Phe Asn Asn Glu Lys Gly Phe
     1               5                  10                  15
   Gly Phe Ile Glu Val Pro Gly Glu Asn Asp Val Phe Val His Phe Ser
                20                  25                  30
   Ala Ile Glu Thr Xaa Gly Phe Lys Ser Leu Glu Glu Gly Gln Lys Val
            35                  40                  45
   Ser Phe Glu Ile Glu Asp Gly Asn Arg
        50                  55
(2) INFORMATION FOR SEQ ID NO: 11:
   (i) SEQUENCE CHARACTERISTICS:
      (A) LENGTH: 171 base pairs
      (B) TYPE: nucleic acid
      (C) STRANDEDNESS: double
      (D) TOPOLOGY: linear
   (ii) MOLECULE TYPE: DNA (genomic)
   (ix) FEATURE:
      (A) NAME/KEY: CDS
      (B) LOCATION:1..171
   (xi) SEQUENCE DESCRIPTION: SEQ ID NO: 11:
   ATG GCA GTA ACA GGA CAA GTA AAA TGG TTT AAC AAC GAA AAA GGC TTC      48
   Met Ala Val Thr Gly Gln Val Lys Trp Phe Asn Asn Glu Lys Gly Phe
            60                  65                  70
   GGT TTC ATC GAA GTT CCA GGC GAA AAC GAY GTA TTC GTA CAY TTC TCT      96
   Gly Phe Ile Glu Val Pro Gly Glu Asn Xaa Val Phe Val Xaa Phe Ser
        75                  80                  85
   GCA ATC GAA ACT GAM GGT TTC AAA TCT CTA GAA GAA GGC CAA AAA GTT     144
   Ala Ile Glu Thr Xaa Gly Phe Lys Ser Leu Glu Glu Gly Gln Lys Val
    90                  95                 100                 105
   AGC TTC GAA ATC GAA GAH GGY AAC CGT                                 171
   Ser Phe Glu Ile Glu Xaa Xaa Asn Arg
                   110
(2) INFORMATION FOR SEQ ID NO: 12:
   (i) SEQUENCE CHARACTERISTICS:
      (A) LENGTH: 57 amino acids
      (B) TYPE: amino acid
      (D) TOPOLOGY: linear
   (ii) MOLECULE TYPE: protein
   (xi) SEQUENCE DESCRIPTION: SEQ ID NO: 12:
   Met Ala Val Thr Gly Gln Val Lys Trp Phe Asn Asn Glu Lys Gly Phe
     1               5                  10                  15
   Gly Phe Ile Glu Val Pro Gly Glu Asn Xaa Val Phe Val Xaa Phe Ser
                20                  25                  30
   Ala Ile Glu Thr Xaa Gly Phe Lys Ser Leu Glu Glu Gly Gln Lys Val
            35                  40                  45
   Ser Phe Glu Ile Glu Xaa Xaa Asn Arg
        50                  55
(2) INFORMATION FOR SEQ ID NO: 13:
   (i) SEQUENCE CHARACTERISTICS:
      (A) LENGTH: 52 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 13:
   TTTTTTWTAC ATGAAGACTA AATAAAATGT ATTTTCGTAG GAGGAAATAA TT            52
(2) INFORMATION FOR SEQ ID NO: 14:
   (i) SEQUENCE CHARACTERISTICS:
      (A) LENGTH: 55 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 14:
   TTTTTTTTAC ATGAAGACTA AATAATAAAA TGTATTTTCT TAGGAGGAAA TAATT         55
(2) INFORMATION FOR SEQ ID NO: 15:
   (i) SEQUENCE CHARACTERISTICS:
      (A) LENGTH: 58 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 15:
   TTTTTTAGAC AGGAAGACTA AAAAAATTAA ATTTGTATTT TCTTAGGAGG AAATAATC 58
(2) INFORMATION FOR SEQ ID NO: 16:
   (i) SEQUENCE CHARACTERISTICS:
      (A) LENGTH: 59 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO: 16:
   TTTTTTAGAY AGGARRACTA AAAAHTTWAA AAWWTGTATT TTMTTAGGAG GAAATAATY 59

## Claims

1. A method of testing for a psychrotrophic *Bacillus* bacterium in a sample, which method comprises determining whether the sample contains a nucleic acid which codes for a *Bacillus* major cold shock protein or major cold shock protein homologue and which has a conserved nucleotide base which is different in psychrotrophic and mesophilic strains of *Bacillus.*

2. A method as claimed in claim 1, for testing for psychrotrophic *Bacillus* in a food sample.

3. A method as claimed in claim 1 or claim 2, which method comprises performing a nucleic acid amplification reaction on a target region of the nucleic acid which codes for a *Bacillus* major cold shock protein or major cold shock protein homologue, which target region comprises the conserved nucleotide base difference.

4. A method as claimed in any one of claims 1 to 3, which method comprises using an oligonucleotide probe which is capable under certain conditions of hybridising specifically to a nucleic acid coding for a psychrotrophic *Bacillus* major cold shock protein or major cold shock protein homologue.

5. A method as claimed in claim 4, wherein the probe is a fluorogenic probe suitable for use in a probe cleavage amplification technique.

6. A method as claimed in any one of claims 1 to 5, wherein the conserved nucleotide base is A at position 4 or T at position 9, or both A at position 4 and T at position 9, in the nucleic acid sequence in Figure 1.

7. A method as claimed in any one of claims 1 to 5, wherein the conserved nucleotide base is T at position 124 in the nucleic acid sequence in Figure 1.

8. A method as claimed in claim 6, using a probe which is complementary to a region of the major cold shock protein nucleic acid sequence for psychrotrophic *Bacillus* shown in Figure 1, or to its reverse complement, which region includes position 4 or position 9 in Figure 1, or both position 4 and position 9.

9. A method as claimed in claim 7, using a probe which is complementary to a region of the major cold shock protein nucleic acid sequence for psychrotrophic *Bacillus* shown in Figure 1, or to its reverse complement, which region includes position 124 in Figure 1.

10. An oligonucleotide probe suitable for use in a method according to any one of claims 4 to 9, comprising at least 15 nucleotides.

11. A method as claimed in claim 3, which method comprises amplifying the target region using a pair of oligonucleotide primers wherein one primer in the pair incorporates the conserved nucleotide base difference at its 3' end.

12. A method as claimed in claim 11, wherein a second primer in the pair incorporates a second conserved nucleotide base difference at its 3' end.

13. A method as claimed in claim 11 or claim 12, wherein the pair of oligonucleotide primers is capable of specifically amplifying psychrotrophic *Bacillus* nucleic acid.

14. A method as claimed in any one of claims 11 to 13, wherein the conserved nucleotide base difference is at position 4 or position 9 or position 124 in the nucleic acid sequence in Figure 1.

15. A pair of oligonucleotide primers suitable for use in a method according to any one of claims 11 to 14.

## Patentansprüche

1. Verfahren zum Untersuchen einer Probe auf ein psychotropes *Bacillus* Bakterium, wobei das Verfahren das Bestimmen umfasst, ob die Probe eine Nucleinsäure enthält, die ein *Bacillus* Hauptkälteschockprotein oder Hauptkälteschockprotein-Homolog kodiert und die eine konservierte Nucleotidbasis hat, die unterschiedliche psychotrope und mesophile *Bacillus* Stämme hat.

2. Verfahren nach Anspruch 1 zum Untersuchen einer Lebensmittelprobe auf psychotropen *Bacillus.*

3. Verfahren nach Anspruch 1 oder Anspruch 2, wobei das Verfahren das Durchführen einer Nucleinsäure-Amplifikationsreaktion an einer Zielregion der Nucleinsäure umfasst, die ein *Bacillus* Hauptkälteschockprotein oder Hauptkälteschockprotein-Homolog kodiert, wobei die Zielregion die konservierte Nucleotidbasisdifferenz umfasst.

4. Verfahren nach einem der Ansprüche 1 bis 3, wobei das Verfahren das Verwenden einer Oligonucleotidsonde umfasst, die unter bestimmten Bedingungen speziell mit einer Nucleinsäure hybridisieren kann, die ein psychotropes *Bacillus* Hauptkälteschockprotein oder Hauptkälteschockprotein-Homolog kodiert.

5. Verfahren nach Anspruch 4, wobei die Sonde eine fluorogene Sonde ist, die zur Verwendung bei einer Sondenspaltungsamplifikationstechnik geeignet ist.

6. Verfahren nach einem der Ansprüche 1 bis 5, wobei die konservierte Nucleotidbasis A bei Position 4 oder T bei Position 9 oder sowohl A bei Position 4 als auch T bei Position 9 in der Nucleinsäuresequenz in Fig. 1 ist.

7. Verfahren nach einem der Ansprüche 1 bis 5, wobei die konservierte Nucleotidbasis T bei Position 124 in der Nucleinsäuresequenz in Fig. 1 ist.

8. Verfahren nach Anspruch 6 unter Verwendung einer Sonde, die komplementär zu einer Region der Hauptkälteschockprotein-Nucleinsäuresequenz für psychotropen *Bacillus* (s. Fig. 1) oder zu ihrem umgekehrten Komplement ist, wobei die Region Position 4 oder Position 9 in Fig. 1 oder sowohl Position 4 als auch Position 9 beinhaltet.

9. Verfahren nach Anspruch 7 unter Verwendung einer Sonde, die komplementär zu einer Region der Hauptkälteschockprotein-Nucleinsäuresequenz für psychotropen *Bacillus* (s. Fig. 1) oder zu ihrem umgekehrten Komplement ist, wobei die Region die Position 124 in Fig. 1 beinhaltet.

10. Oligonucleotidsonde, die zur Verwendung in einem Verfahren nach einem der Ansprüche 4 bis 9 geeignet ist, umfassend wenigstens 15 Nucleotide.

11. Verfahren nach Anspruch 3, wobei das Verfahren das Amplifizieren der Zielregion mit einem Paar Oligonucleotid-Primer umfasst, wobei ein Primer in dem Paar die konservierte Nucleotidbasisdifferenz an seinem 3'-Ende einschließt.

12. Verfahren nach Anspruch 11, wobei ein zweiter Primer in dem Paar eine zweite konservierte Nucleotidbasisdifferenz an seinem 3'-Ende einschließt.

13. Verfahren nach Anspruch 11 oder Anspruch 12, wobei das Oligonucleotid-Primer-Paar in der Lage ist, speziell psychotrope *Bacillus* Nucleinsäure zu amplifizieren.

14. Verfahren nach einem der Ansprüche 11 bis 13, wobei die konservierte Nucleotidbasisdifferenz bei Position 4 oder Position 9 oder Position 124 in der Nucleinsäuresequenz in Fig. 1 ist.

15. Oligonucleotid-Primer-Paar, das zur Verwendung in einem Verfahren nach einem der Ansprüche 11 bis 14 geeignet ist.

## Revendications

1. Méthode d'essai pour une bactérie *Bacillus* psychrotrophe dans un échantillon, laquelle méthode comprend la détermination de la présence ou non dans l'échantillon d'un acide nucléique qui code pour une protéine de choc froid majeure ou un homologue de protéine de choc froid majeur de *Bacillus* et qui a une base nucléotidique conservée qui est différente dans les souches psychrotrophes et mésophiles de *Bacillus*.

2. Méthode selon la revendication 1, pour un essai pour un *Bacillus* psychrotrophe dans un échantillon alimentaire.

3. Méthode selon la revendication 1 ou la revendication 2, laquelle méthode comprend la réalisation d'une réaction d'amplification d'un acide nucléique sur une région cible de l'acide nucléique qui code pour une protéine de choc froid majeure ou un homologue de protéine de choc froid majeur de *Bacillus*, laquelle région cible comprend la différence de base nucléotidique conservée.

4. Méthode selon l'une quelconque des revendications 1 à 3, laquelle méthode comprend l'utilisation d'une sonde oligonucléotidique qui est capable, dans certaines conditions, d'hybridation spécifique avec un acide nucléique codant pour une protéine de choc froid majeure ou un homologue de protéine de choc froid majeur de *Bacillus* psychrotrophe.

5. Méthode selon la revendication 4, dans laquelle la sonde est une sonde fluorogénique convenable pour une utilisation dans une technique d'amplification par clivage de sonde.

6. Méthode selon l'une quelconque des revendications 1 à 5, dans laquelle la base nucléotidique conservée est A en position 4 ou T en position 9, ou A en position 4 ainsi que T en position 9, dans la séquence d'acide nucléique de la Figure 1.

7. Méthode selon l'une quelconque des revendications 1 à 5, dans laquelle la base nucléotidique conservée est T en position 124 dans la séquence d'acide nucléique de la Figure 1.

8. Méthode selon la revendication 6, utilisant une sonde qui est complémentaire d'une région de la séquence d'acide nucléique de la protéine de choc froid majeure pour le *Bacillus* psychrotrophe illustrée à la Figure 1, ou de son complément réverse, laquelle région inclut la position 4 ou la position 9 à la Figure 1, ou la position 4 ainsi que la position 9.

9. Méthode selon la revendication 7, utilisant une sonde qui est complémentaire d'une région de la séquence d'acide nucléique de la protéine de choc froid majeure pour le *Bacillus* psychrotrophe illustrée à la Figure 1, ou de son complément réverse, laquelle région inclut la position 124 à la Figure 1.

10. Sonde oligonucléotidique convenable pour une utilisation dans une méthode selon l'un quelconque des revendications 4 à 9, comprenant au moins 15 nucléotides.

11. Méthode selon la revendication 3, laquelle méthode comprend l'amplification de la région cible à l'aide d'une paire d'amorces oligonucléotidiques, dans laquelle l'une des amorces de la paire incorpore la différence de base nucléotidique conservée à son extrémité 3'.

12. Méthode selon la revendication 11, dans laquelle une deuxième amorce de la paire incorpore une deuxième différence de base nucléotidique conservée à son extrémité 3'.

13. Méthode selon la revendication 11 ou la revendication 12, dans laquelle la paire d'amorces oligonucléotidiques est capable d'amplifier spécifiquement l'acide nucléique de *Bacillus* psychrotrophe.

14. Méthode selon l'une quelconque des revendications 11 à 13, dans laquelle la différence de base nucléotidique conservée est en position 4, ou en position 9, ou en position 124 dans la séquence d'acide nucléique de la Figure 1.

15. Paire d'amorces oligonucléotidiques convenable pour une utilisation dans une méthode selon l'une quelconque des revendications 11 à 14.
